(19) **Europäisches Patentamt**
**European Patent Office**
**Office européen des brevets**

(11) **EP 4 285 989 A1**

(12) **EUROPEAN PATENT APPLICATION**
published in accordance with Art. 153(4) EPC

(43) Date of publication:
**06.12.2023 Bulletin 2023/49**

(21) Application number: **22775686.3**

(22) Date of filing: **23.03.2022**

(51) International Patent Classification (IPC):
**A61P 1/14** (2006.01)   **A61P 31/04** (2006.01)
**A61P 31/12** (2006.01)   **A61P 37/04** (2006.01)
**A61K 35/741** (2015.01)   **A61K 31/724** (2006.01)
**A23L 33/135** (2016.01)   **A23L 33/21** (2016.01)

(52) Cooperative Patent Classification (CPC):
**A23L 33/135; A23L 33/21; A61K 31/724;**
**A61K 35/741; A61P 1/14; A61P 31/04;**
**A61P 31/12; A61P 37/04**

(86) International application number:
**PCT/JP2022/013542**

(87) International publication number:
**WO 2022/202904 (29.09.2022 Gazette 2022/39)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **25.03.2021   JP 2021051428**

(71) Applicants:
• **Cyclochem Bio Co., Ltd.**
  **Kobe-shi, Hyogo 650-0047 (JP)**
• **Ace Bio Product Co., Ltd.**
  **Tokyo 101-0031 (JP)**

(72) Inventors:
• **TERAO Keiji**
  **Kobe-shi, Hyogo 650-0047 (JP)**
• **FURUNE Takahiro**
  **Kobe-shi, Hyogo 650-0047 (JP)**
• **UENO Chihiro**
  **Kobe-shi, Hyogo 650-0047 (JP)**
• **CHIKAMOTO Keita**
  **Kobe-shi, Hyogo 650-0047 (JP)**
• **HASEGAWA Risa**
  **Kobe-shi, Hyogo 650-0047 (JP)**
• **TAKIZAWA Jo**
  **Tokyo 101-0031 (JP)**

(74) Representative: **Forstmeyer, Dietmar et al**
**Boeters & Lieck**
**Oberanger 32**
**80331 München (DE)**

(54) **INTESTINAL TRACT FUNCTION-IMPROVING AGENT**

(57)    A safe and useful novel agent and the like are provided by investigating the effect of a cyclodextrin (CD) itself on a butyric acid bacterium, and combining a CD with at least one of a butyric acid bacterium, butyric acid, or acetic acid. As a safe and useful novel agent, an intestinal tract function-improving agent, an immunoenhancing agent, an anti-bacterial agent, and a hydrogen generating agent and/or an anti-oxidizing agent, including, as active ingredients: a cyclodextrin; and at least one of a butyric acid bacterium, butyric acid, or acetic acid are provided. Further, in the invention, for example, a proliferation promoting agent of a butyric acid bacterium, a production promoting agent of butyric acid, and a production promoting agent of acetic acid, including, as an active ingredient: a cyclodextrin, a method of producing butyric acid or a method of producing acetic acid, including the step of: applying a cyclodextrin to a butyric acid bacterium can also be provided.

[FIG. 1]

**p< 0.01 compared with α-CD treatment.

**Description**

TECHNICAL FIELD

The present invention relates to an intestinal tract function-improving agent, including, as active ingredients: a cyclodextrin (hereinafter may be simply referred to as CD); and at least one of a butyric acid bacterium, butyric acid, or acetic acid. The invention further relates to an immunoenhancing agent, an infection preventive agent for a virus or a microbe, an anti-bacterial agent, and a hydrogen generating agent and/or an anti-oxidizing agent, including, as active ingredients: a CD; and at least one of a butyric acid bacterium, butyric acid, or acetic acid, and further relates to, for example, a proliferation promoting agent of a butyric acid bacterium,

BACKGROUND ART

Human enteric bacteria degrade dietary fiber and indigestible oligosaccharides in the intestine and produce short-chain fatty acids (hereinafter may be simply referred to as SCFAs) that provide various health-promoting effects to the host. Butyric acid, which is one of the SCFAs, is known to be produced from a butyric acid bacterium (Clostridium butyricum) used as a probiotic raw material and the like, and be an energy source or exhibit an antiinflammatory effect (see, for example, Non-Patent Document 1). It has also been suggested that, for example, a butyric acid bacterium and butyric acid have an infection protective action against pathogenic Escherichia coli, and the expression of genes related to viral infection may be regulated by butyric acid to reduce the susceptibility to viruses (see, for example, Non-Patent Documents 2 and 3).

For the purpose of utilizing these actions of SCFAs such as butyric acid for the treatment of gastrointestinal disorders such as colitis, inflammatory bowel disease, and irritable bowel syndrome, a technique of using a CD inclusion complex as a delivery vehicle has been developed. It has been disclosed that an inclusion complex in which butyric acid, propionic acid, and acetic acid are included in $\alpha$-cyclodextrin (hereinafter may be simply referred to as $\alpha$-CD) was as used for gastrointestinal treatment (see, for example, Patent Document 1).

It is known that administering a butyric acid bacterium to an intestinal bacteria-free mouse has an effect of increasing intestinal IgA (see, for example, Non-Patent Document 4), and a technique has also been disclosed for making a butyric acid bacterium odorless by mixing a butyric acid bacterium with a CD, which is a cyclic dextrin, and drying the mixture when a butyric acid bacterium itself is used as an intestinal regulator or a nutrient (see, for example, Patent Document 2). However, this technique is intended to deliver butyric acid to the gastrointestinal tract and to make butyric acid odorless, and the effects of a CD itself on gastrointestinal tract are not suggested or studied on either of the techniques.

In Patent Document 3, a butyric acid bacterium and a CD are listed as one of the components that improve the intestinal environment to provide a function-enhancing composition that improves the bioavailability of the active ingredient of a general food, a health-promoting food, or a dietary supplement. However, the effect of a butyric acid bacterium and a CD themselves and the specific effect of combining them on improving intestinal tract function have not been studied, and they are just listed.

Thus, the present inventors have investigated the effect of a CD itself on a butyric acid bacterium, studied the effect of combining a CD with a butyric acid bacterium and/or butyric acid, and tried to provide a safe and useful novel agent.

Further, the present inventors have also focused on hydrogen produced when a butyric acid bacterium assimilate a carbon source. It is known that, for example, hydrogen produced in the digestive tract has an anti-oxidant action and eliminates hydroxyl radicals having high reactivity among reactive oxygen species (see, for example, Non-Patent Document 5). Thus, the present inventors have also studied the effect of combining a CD with a butyric acid bacterium and/or butyric acid on the production of hydrogen, and tried to provide a novel agent.

CITATION LIST

PATENT LITERATURE

Patent Document 1: JP2019-512019A
Patent Document 2: JPS54-005092A
Patent Document 3: JP2005-034135A

NON PATENT LITERATURE

Non-Patent Document 1: Hiroshi Oyaizu (2020). Todai no biseibutuhakase ga oshieru corona ni korosarenai tatta hitotuno houhou, JIYUKOKUMINSHA

Non-Patent Document 2: Motomichi Takahashi et. al., (2004). The effect of probiotic treatment with Clostridium butyricum on enterohemorrhagic Escherichia coli O157:H7 infection in mice FEMS Immunology and Medical Microbiology, 41, 219-226.

Non-Patent Document 3: Jing Li et. al., (2021). Butyrate Regulates COVID-19-Relevant Genes in Gut Epithelial Organoids From Normotensive Rats, 77, e13-e16.

Non-Patent Document 4: Taka-ichi Murayama et. ai., (1995). Effects of orally administrated Clostridium butyricum MIYAIRAI 588 on mucosal immunity in mice. Vererinary Immunology and Immunopathology, 48, 333-342.

Non-Patent Document 5: Ohsawa I, Ishikawa M, Takahashi K et al., (2007). Hydrogen acts as a therapeutic antioxidant by selectively reducing cytotoxic oxygen radicals. Nat Med 13, 688-694.

## SUMMARY OF INVENTION

### TECHNICAL PROBLEM

An object of the invention is to study the effect of a CD itself on a butyric acid bacterium, and to provide a safe and useful novel agent and the like by combining a CD with at least one of a butyric acid bacterium, butyric acid, or acetic acid.

### SOLUTION TO PROBLEM

As a result of intensive studies to solve the problems, the present inventors have found that a CD promotes the proliferation of a butyric acid bacterium and the production of butyric acid and acetic acid. Also, along with these, the present inventors have found that a combination of a CD and at least one of a butyric acid bacterium, butyric acid, or acetic acid has effects such as improvement of intestinal tract functions, enhancement of immune functions, infection prevention for viruses or microbes, an anti-bacterial action against harmful microbes such as Staphylococcus aureus, and a hydrogen generating action and/or an anti-oxidant action, and completed the invention.

That is, the invention relates to an intestinal tract function-improving agent and the like shown in (1) to (15) below.

(1) An intestinal tract function-improving agent containing a CD; and at least one of a butyric acid bacterium, butyric acid, or acetic acid as active ingredients.

(2) An immunoenhancing agent containing a CD and at least one of a butyric acid bacterium, butyric acid, or acetic acid as active ingredients.

(3) An infection preventive agent for a virus or a microbe containing a CD and at least one of a butyric acid bacterium, butyric acid, or acetic acid as active ingredients.

(4) An anti-bacterial agent containing a CD and at least one of a butyric acid bacterium, butyric acid, or acetic acid as active ingredients.

(5) A hydrogen generating agent and/or an anti-oxidizing agent containing a CD and at least one of a butyric acid bacterium, butyric acid, or acetic acid as active ingredients.

(6) A proliferation promoting agent of a butyric acid bacterium containing a CD as an active ingredient.

(7) A production promoting agent of butyric acid containing a CD as an active ingredient.

(8) A production promoting agent of acetic acid containing a CD as an active ingredient.

(9) A method of producing butyric acid containing the step of applying a CD to a butyric acid bacterium.

(10) A method of producing acetic acid containing the step of applying a CD to a butyric acid bacterium.

(11) A method of improving an intestinal tract function, including the step of: administering a CD and at least one of a butyric acid bacterium, butyric acid, or acetic acid.

(12) A method of immunoenhancing, including the step of: administering a CD and at least one of a butyric acid bacterium, butyric acid, or acetic acid.

(13) A method of preventing infection with a virus or a microbe, including the step of: administering a CD and at least one of a butyric acid bacterium, butyric acid, or acetic acid.

(14) A method of imparting anti-bacterial action, including the step of: administering a CD and at least one of a butyric acid bacterium, butyric acid, or acetic acid.

(15) A method of generating hydrogen and/or a method of imparting an anti-oxidant action, including the step of: administering a CD and at least one of a butyric acid bacterium, butyric acid, or acetic acid.

ADVANTAGEOUS EFFECTS OF INVENTION

According to the invention, it has become possible to provide a proliferation promoting agent of a butyric acid bacterium, a production promoting agent of butyric acid, and a production promoting agent of acetic acid, including, as an active ingredient, a CD, and also to provide a method of producing butyric acid, or a method of producing acetic acid. Further, it has become possible to provide an agent that can exhibit an action of improving intestinal tract functions, an action of enhancing immune functions, an action of infection prevention for viruses or microbes, an anti-bacterial action against harmful bacteria, a hydrogen generating action and/or an anti-oxidant action and the like by combining a CD with at least one of a butyric acid bacterium, butyric acid, or acetic acid.

Each agent obtained by the invention can be used for various uses. For example, it can be used as it is as foods, health foods, feed, quasi-drugs, pharmaceuticals and the like, or used as an active ingredient of an antiinflammatory agent, an immunostimulant agent and the like. The agent is also expected to have beneficial effects on beauty and health when taken as a supplement.

BRIEF DESCRIPTION OF DRAWINGS

[Fig. 1] A figure showing the effects on proliferation promoting of a butyric acid bacterium (Example 6).
[Fig. 2] A figure showing the effects on production promoting of butyric acid (Example 6).
[Fig. 3] A figure showing the effects on production promoting of acetic acid (Example 6).
[Fig. 4] A figure showing the effects on proliferation inhibiting of Staphylococcus aureus (Example 7).
[Fig. 5] A figure showing an increased amount of gas (Example 8).
[Fig. 6] A figure showing a hydrogen production amount (Example 8).

DESCRIPTION OF EMBODIMENTS

The "intestinal tract function-improving agent", "immunoenhancing agent", "infection preventive agent for a virus or a microbe", "anti-bacterial agent", and "hydrogen generating agent and/or an anti-oxidizing agent" of the invention all refer to an agent that exhibits the following effects on the animals that have taken the agent when taken by animals such as humans, mice, rats, rabbits, dogs, cats, horses, sheep, monkeys, cows, pigs, and chickens.

That is, examples thereof include the effect of improving intestinal tract functions, the effect of enhancing immune functions, and the effect of preventing infection with harmful viruses such as coronavirus, influenza virus or upper respiratory tract infectionrelated virus and harmful microbes such as Staphylococcus aureus and Escherichia coli. Examples thereof also include the effect of the proliferation inhibiting of these microbes, the effect of inhibiting the action of toxins and the like produced by these microbes, the effect of generating hydrogen in the digestive tract and the effect of promoting the production of hydrogen, and the effect of exhibiting an anti-oxidant action by the generated or produced hydrogen.

Such "intestinal tract function-improving agent", "immunoenhancing agent", "infection preventive agent for a virus or a microbe", "anti-bacterial agent", and "hydrogen generating agent and/or an anti-oxidizing agent" of the invention can be all an agent including, as active ingredients, a CD and at least one of a butyric acid bacterium, butyric acid, or acetic acid. The agent can be an agent further including, in addition to these, other components such as useful components and components necessary for maintaining the dosage form.

Further, the "proliferation promoting agent of a butyric acid bacterium", "production promoting agent of butyric acid", and "production promoting agent of acetic acid" of the invention all refer to an agent that exhibits the effect of proliferation promoting of a butyric acid bacterium itself and the production of butyric acid and acetic acid by applying a CD to a butyric acid bacterium.

Such "proliferation promoting agent of a butyric acid bacterium", "production promoting agent of butyric acid", and "production promoting agent of acetic acid" of the invention can be all an agent including a CD as an active ingredient, and can be an agent further including other components such as useful components and components necessary for maintaining the dosage form.

The "method of producing butyric acid" or "method of producing acetic acid" of the invention can be a method "including the step of: applying a CD to a butyric acid bacterium", and can be a production method further including other steps useful for the production of butyric acid.

The CD that can be used as an active ingredient of the "intestinal tract function-improving agent", "immunoenhancing agent", "infection preventive agent for a virus or a microbe", "anti-bacterial agent", "hydrogen generating agent and/or an anti-oxidizing agent", "proliferation promoting agent of a butyric acid bacterium", "production promoting agent of butyric acid", and "production promoting agent of acetic acid" of the invention and the CD that can be used in the "method of producing butyric acid" or "method of producing acetic acid" can be a CD that can be safely taken by animals, and can be commercially available one or independently prepared one.

Examples of such CD include α-CD, β-CD, γ-CD, maltosyl-α-CD, maltosyl-β-CD, and maltosyl-γ-CD. One type of these CDs can be used, or two or more types thereof can be used in combination as an active ingredient.

Examples of commercially available α-CD include Pure Fiber (COSANA Corporation) and indigestible α oligosaccharides (COSANA Corporation).

Further, as a butyric acid bacterium that can be used as an active ingredient of "intestinal tract function-improving agent", "immunoenhancing agent", "infection preventive agent for a virus or a microbe", "anti-bacterial agent", and "hydrogen generating agent and/or an anti-oxidizing agent", any one that can be safely taken by animals can be used, and examples thereof include Clostridium butyricum M-II 588 strain (ACE BIO PRODUCT CO., LTD), Clostridium butyricum NT strain (NITTO PHARMACEUTICAL INDUSTRIES, LTD.), and Clostridium butyricum TO-A strain (TOA SHINYAKU CO., LTD.)

Examples of butyric acid and acetic acid include butyric acid and acetic acid produced by these butyric acid bacteria and commercially available butyric acid and acetic acid.

The "intestinal tract function-improving agent", "immunoenhancing agent", "infection preventive agent for a virus or a microbe", "anti-bacterial agent", "hydrogen generating agent and/or an anti-oxidizing agent", and "proliferation promoting agent of a butyric acid bacterium" of the invention can be administered in an amount that is effective in the animals that have taken them.

For example, when the animal that takes them is a human, the amount of the CD contained in each agent and taken is preferably about 0.001 to 10 g/day, and particularly preferably about 2 to 10 g/day. Similarly, the amount of butyric acid bacteria is preferably about 1 to $10^{10}$ CFU/day, and particularly preferably about $10^6$ to $10^8$ CFU/day. The amount of butyric acid is preferably about 0.00001 to 5 g/day, and particularly preferably about 0.1 to 2 g/day. Further, the amount of acetic acid is preferably about 0.00001 to 5 g/day, and particularly preferably about 0.1 to 3 g/day.

Hereinafter, the invention will be described in detail with reference to Examples, but the invention is not limited to those shown in Examples.

EXAMPLES

Samples used in Examples of the invention are shown below.

<Sample>

1. Carbohydrate

    1) α-CD (CAVAMAXW6 (registered trademark) Food, CycloChem Bio Co., Ltd.)
    2) Galactooligosaccharide (GOS) (galactooligosaccharide, FUJIFILM Wako Pure Chemical Corporation)
    3) Fructooligosaccharide (FOS) (fructooligosaccharide, FUJIFILM Wako Pure Chemical Corporation)
    4) Indigestible dextrin (ID) (Fibersol (registered trademark) 2, Matsutani Chemical Industry Co., Ltd.)
    5) Isomaltodextrin (IMD) (Fibryxa (registered trademark), Hayashibara Co., Ltd.)
    6) Inulin (INL) (Inulin, FUJIFILM Wako Pure Chemical Corporation)
    7) Polydextrose (PDX) (STA-LITE (registered trademark) III, KOYO MERCANTILE CO., LTD.)
    8) Partially Hydrolyzed Guar Gum (PHGG) (Sunfiber (registered trademark), Taiyo-labo TL1)
    9) Lactosucrose (LS) (Oligonookage (registered trademark) LS-90P, ENSUIKO Sugar Refining Co., Ltd.)

2. Butyric acid bacterium

    Clostridium butyricum M-II588 (ACE BIO PRODUCT CO., LTD) Basal medium: GAM bouillon "Nissui" (Nissui Pharmaceutical Co., Ltd.)
    Sugar-free medium for assimilation test: Semi solid medium for GAM glycolysis "Nissui" (Nissui Pharmaceutical Co., Ltd.)

This medium was added to cold water, the mixture was stirred well, then the agar was removed by filtration, and then the medium was autoclaved and used.

3. Staphylococcus aureus

Staphylococcus aureus IFO 12732
Medium: GAM bouillon "Nissui" (Nissui Pharmaceutical Co., Ltd.)

[Example 1]

Method for producing intestinal tract function-improving agent

A combination of $\alpha$-CD and at least one of a butyric acid bacterium, butyric acid, or acetic acid was used as an intestinal tract function-improving agent.

[Example 2]

Method for producing immunoenhancing agent

A combination of $\alpha$-CD and at least one of a butyric acid bacterium, butyric acid, or acetic acid was used as an immunoenhancing agent.

[Example 3]

Method for producing infection preventive agent for a virus or a microbe

A combination of $\alpha$-CD and at least one of a butyric acid bacterium, butyric acid, or acetic acid was used as an infection preventive agent for a virus or a microbe.

[Example 4]

Method for producing anti-bacterial agent

A combination of $\alpha$-CD and at least one of a butyric acid bacterium, butyric acid, or acetic acid was used as an anti-bacterial agent.

[Example 5]

Method for producing hydrogen generating agent and/or an anti-oxidizing agent

A combination of $\alpha$-CD and at least one of a butyric acid bacterium, butyric acid, or acetic acid was used as a hydrogen generating agent and/or an anti-oxidizing agent.

[Example 6]

Study of the proliferation promoting effect on a butyric acid bacterium and the production promoting effect of butyric acid and/or acetic acid

A butyric acid bacterium colony was inoculated into the basal medium, and cultured at 37°C for 20 hours under an anaerobic condition (preculture). The preculture solution was centrifuged (3,700 rpm, 8 min), and the pellet containing butyric acid bacteria were resuspended in a sugar-free medium for assimilation test (turbidity at 660 nm was about 1.2). In a new sugar-free medium for assimilation test, 1 w/v% of each of various carbohydrates was dissolved, a resuspension containing butyric acid bacteria was added at 1 v/v%, and culture was performed in a sealed bag under an anaerobic condition at 37°C (main culture). The turbidity change ($\Delta$OD) at 660 nm 20 hours after the start of culture was checked, and whether each of various added carbohydrates was assimilated and butyric acid bacteria grew was evaluated.

The culture solution after the culture was collected, the pH was measured using Two Band pH Test Paper (DUOTEST), the amounts of butyric acid and acetic acid contained in the culture solution were measured using GC-FID after derivatization with N-(tert-butyldimethylsilyl)-N-methyltrifluoroacetamide (MTBSTFA) according to Parker et al. (S. G. Parker, et al., Jounal of Functional Foods, 31, 266-273 (2017)), and whether the production of butyric acid and acetic acid was promoted was evaluated.

The test results are shown as mean $\pm$ standard error of three samples. Dunnett's test was performed for statistical processing using statistical analysis software, Pharmaco Analyst I (Ver. 13.1.1; Three S Japan, Tokyo, Japan).

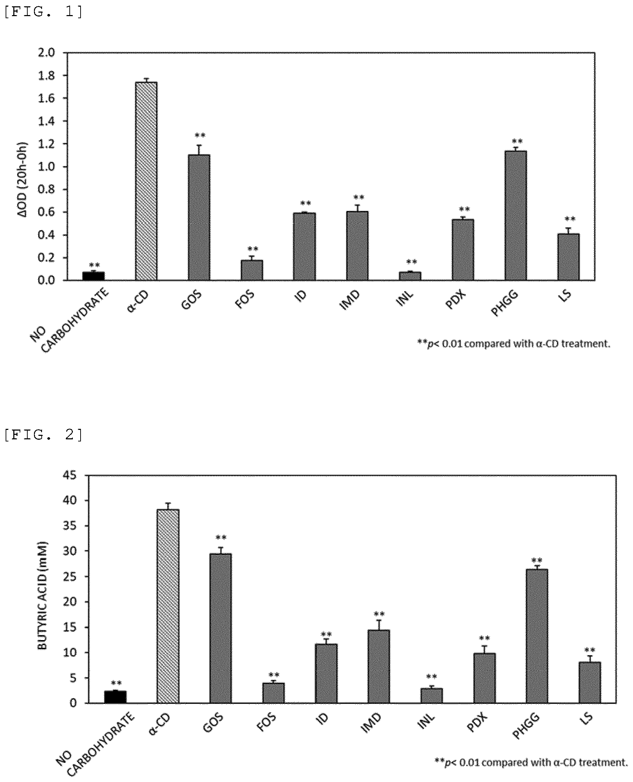

As a result, it was confirmed that addition of α-CD significantly promoted the proliferation of butyric acid bacteria and the production of butyric acid and acetic acid compared to other carbohydrates as shown in FIGS. 1 to 3. Further, a decrease in pH was confirmed as shown in Table 1.

Thus, the results suggest that intake of α-CD significantly increases the number of butyric acid bacteria in the intestinal bacterial flora and also increases the amount of butyric acid and acetic acid, which works to improve the intestinal tract function.

[Table 1]

|  | pH After culture for 20 hours |
|---|---|
| No carbohydrate | $6.8 \pm 0.0$** |
| α-CD | $4.7 \pm 0.0$ |
| GOS | $5.5 \pm 0.3$** |
| FOS | $6.6 \pm 0.2$** |
| ID | $6.1 \pm 0.1$** |
| IMD | $6.2 \pm 0.0$** |
| INL | $6.8 \pm 0.0$** |
| PDX | $6.3 \pm 0.2$** |
| PHGG | $5.5 \pm 0.1$** |
| LS | $6.3 \pm 0.1$** |
| **$p<0.01$ compared with α-CD treatment |  |

[Example 7]

Study of anti-bacterial action

Culture of Staphylococcus aureus alone, Staphylococcus aureus and 0.5 w/v% α-CD in coexistence, Staphylococcus aureus and butyric acid bacteria in coexistence, or Staphylococcus aureus and butyric acid bacteria in coexistence with 0.5 w/v% α-CD added was performed at 37°C under an anaerobic condition, and the number of Staphylococcus aureus was counted 20 hours after the start of culture. The amount of Staphylococcus aureus added was $7.5 \times 10^2$ CFU/mL as the final concentration, and the amount of butyric acid bacteria added was $9.4 \times 10^6$ CFU/mL as the final concentration. The number of Staphylococcus aureus was measured by the dilution plate technique, that is, spreading a moderately diluted culture solution on an agar medium, culturing it at 37°C under an aerobic condition, and counting the number of colonies formed. The test results are shown as mean ± standard error of three samples.

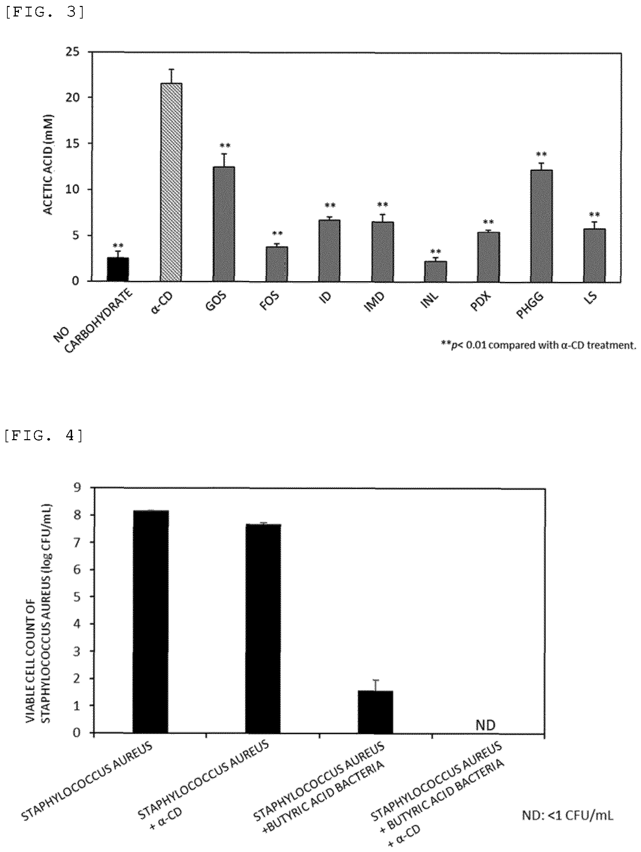

As a result, as shown in FIG. 4, it was shown that α-CD alone showed almost no proliferation inhibiting effect, but the proliferation of Staphylococcus aureus was significantly inhibited by adding butyric acid bacteria, and the proliferation of Staphylococcus aureus was completely inhibited by adding α-CD to butyric acid bacteria.

Thus, from this result, it was confirmed that the combination of α-CD and butyric acid bacteria exhibited strong anti-bacterial activity against harmful bacteria such as Staphylococcus aureus.

[Example 8]

Study of hydrogen generating action and/or anti-oxidant action

A butyric acid bacterium colony was inoculated into the basal medium, and cultured at 37°C for 20 hours under an anaerobic condition (preculture). In 100 mL of a sugar-free medium for assimilation test, 1 w/v% of each of various carbohydrates was dissolved, the preculture solution was added at 5 v/v%, and culture was performed in a sealed bag under an anaerobic condition at 37°C for 24 hours (main culture).

The change in volume from the beginning of the main culture to the end of culture was checked and the increased amount of gas was calculated. The hydrogen concentration in gas after the culture was also measured using a hydrogen detector tube (KOMYO RIKAGAKU KOGYO K.K.) From the measured hydrogen concentration and increased amount of gas, the amount of hydrogen produced (mL) by the culture was calculated by the following formula.

The test results are shown as mean ± standard error of three to five samples. Dunnett's test was performed for statistical processing using statistical analysis software, Pharmaco Analyst I (Ver. 13.1.1; Three S Japan, Tokyo, Japan).

[Formula 1]

```
Hydrogen production amount (mL) = increased amount of gas

(mL) × hydrogen concentration (%) × 1/100
```

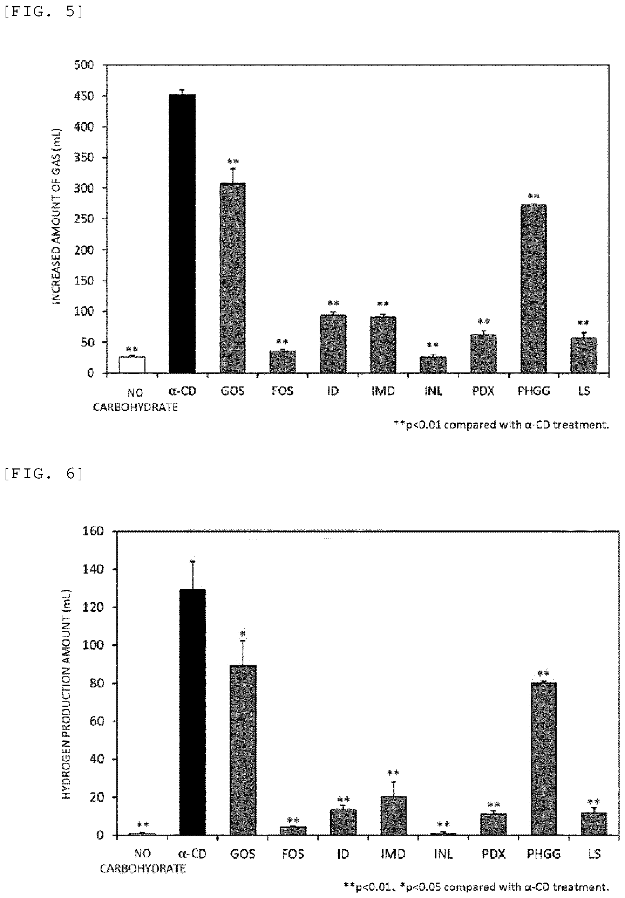

As a result, as shown in FIGS. 5 and 6, it was confirmed that the addition of α-CD significantly increased gas compared to other carbohydrates and hydrogen was generated. When FOS or INL was added as other carbohydrates, there was little increase in gas and hydrogen generation, and when ID, IMD, PDX, or LS was added, increase in gas and hydrogen generation were observed, but the amounts were very little. When GOS or PHGG was added, the increased amount of gas and the amount of hydrogen generation increased, but the significant effect as when α-CD was added was not observed. Thus, from these results, it was confirmed that intake of α-CD causes hydrogen generation in butyric acid bacteria in the intestinal bacterial flora.

It is known that hydrogen has an anti-oxidant action, and acts on, for example, the elimination of hydroxyl radicals that induce diseases such as arteriosclerosis, myocardial infarction, and cancer by oxidation of biological components such as proteins, lipids, and nucleic acids. Thus, it was suggested that hydrogen generation occurs by intake of α-CD, which results in the exhibition of a high anti-oxidant action.

INDUSTRIAL APPLICABILITY

According to the invention, it has become possible to provide a proliferation promoting agent of a butyric acid bacterium, a production promoting agent of butyric acid, and a production promoting agent of acetic acid, including, as an active ingredient, a CD, and also to provide a method of producing butyric acid, or a method of producing acetic acid. Further, it has become possible to provide an agent that can exhibit an action of improving intestinal tract functions, an action of enhancing immune functions, an anti-bacterial action against harmful bacteria, a hydrogen generating action and/or an anti-oxidant action and the like by combining a CD with at least one of a butyric acid bacterium, butyric acid, or acetic acid.

Each agent obtained by the invention can be used for various uses. For example, it can be used as it is as foods, health foods, feed, quasi-drugs, pharmaceuticals and the like, or used as an active ingredient of an antiinflammatory agent, an immunostimulant agent and the like. The agent is also expected to have beneficial effects on beauty and health when taken as a supplement.

## Claims

1. An intestinal tract function-improving agent containing a cyclodextrin and at least one of a butyric acid bacterium, butyric acid, or acetic acid as active ingredients.

2. An immunoenhancing agent containing a cyclodextrin and at least one of a butyric acid bacterium, butyric acid, or acetic acid as active ingredients.

3. An infection preventive agent for a virus or a microbe containing a cyclodextrin and at least one of a butyric acid bacterium, butyric acid, or acetic acid as active ingredients.

4. An anti-bacterial agent containing a cyclodextrin and at least one of a butyric acid bacterium, butyric acid, or acetic acid as active ingredients.

5. A hydrogen generating agent and/or an anti-oxidizing agent containing a cyclodextrin and at least one of a butyric acid bacterium, butyric acid, or acetic acid as active ingredients.

6. A proliferation promoting agent of a butyric acid bacterium containing a cyclodextrin as an active ingredient.

[FIG. 1]

**p< 0.01 compared with α-CD treatment.

[FIG. 2]

**p< 0.01 compared with α-CD treatment.

[FIG. 3]

**p< 0.01 compared with α-CD treatment.

[FIG. 4]

ND: <1 CFU/mL

[FIG. 5]

**p<0.01 compared with α-CD treatment.

[FIG. 6]

**p<0.01、*p<0.05 compared with α-CD treatment.

## INTERNATIONAL SEARCH REPORT

| International application No. |
| --- |
| **PCT/JP2022/013542** |

### A. CLASSIFICATION OF SUBJECT MATTER

*A61P 1/14*(2006.01)i; *A61P 31/04*(2006.01)i; *A61P 31/12*(2006.01)i; *A61P 37/04*(2006.01)i; *A61K 35/741*(2015.01)i; *A61K 31/724*(2006.01)i; *A23L 33/135*(2016.01)i; *A23L 33/21*(2016.01)i

FI: A23L33/135; A23L33/21; A61K35/741; A61P1/14; A61P37/04; A61P31/12; A61P31/04; A61K31/724

According to International Patent Classification (IPC) or to both national classification and IPC

### B. FIELDS SEARCHED

Minimum documentation searched (classification system followed by classification symbols)

A61P1/14; A61P31/04; A61P31/12; A61P37/04; A61K35/741; A61K31/724; A23L33/135; A23L33/21

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2019-512019 A (CZAP RESEARCH AND DEVELOPMENT, LLC) 09 May 2019 (2019-05-09)<br>claims 1, 13, 16, 17, paragraphs [0065]-[0067], [0085] | 1 |
| A | | 2-6 |
| X | 生田直子, 篠原涼平, 佐々木大介, 佐々木建吾, 培養系ヒト腸内細菌叢モデルKUHIMM を利用した食物繊維の影響評価, ニューフードインダストリー, 2018, vol. 60, no. 5, pages 37-43, (New Food Industry.), non-official translation (IKUTA, Naoko, SHINOHARA, Ryohei, SASAKI, Daisuke, SASAKI, Kengo. Evaluation of the effect of dietary fiber using the cultured human intestinal flora model KUHIMM.)<br>page 37, left column, page 38, right column, page 39, right column, page 40, left column, fig. 3(c) | 1, 6 |
| A | | 2-5 |

☐ Further documents are listed in the continuation of Box C.   ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 June 2022** | **05 July 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/013542**

| Patent document cited in search report | Publication date (day/month/year) | Patent family member(s) | Publication date (day/month/year) |
|---|---|---|---|
| JP 2019-512019 A | 09 May 2019 | US 2017/0224841 A1 claims 1, 13, 16, 17, paragraphs [0060]-[0062] EP 3892303 A1 CN 108883125 A KR 10-2019-0005823 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019512019 A
- JP S54005092 A
- JP 2005034135 A


**Non-patent literature cited in the description**

- **HIROSHI OYAIZU.** Todai no biseibutuhakase ga oshieru corona ni korosarenai tatta hitotuno houhou. *JIYUKOKUMINSHA,* 2020
- **MOTOMICHI TAKAHASHI.** The effect of probiotic treatment with Clostridium butyricum on enterohemorrhagic. *Escherichia coli O,* 2004, vol. 157, H7
- *FEMS Immunology and Medical Microbiology,* vol. 41, 219-226
- **JING LI.** Butyrate Regulates COVID-19-Relevant Genes in Gut. *Epithelial Organoids From Normotensive Rats,* 2021, vol. 77, e13-e16
- **TAKA-ICHI MURAYAMA.** Effects of orally administrated Clostridium butyricum MIYAIRAI 588 on mucosal immunity in mice. *Vererinary Immunology and Immunopathology,* 1995, vol. 48, 333-342
- **OHSAWA I ; ISHIKAWA M ; TAKAHASHI K et al.** Hydrogen acts as a therapeutic anti-oxidant by selectively reducing cytotoxic oxygen radicals. *Nat Med,* 2007, vol. 13, 688-694
- **S. G. PARKER et al.** *Jounal of Functional Foods,* 2017, vol. 31, 266-273